# EUROPÄISCHE PATENTSCHRIFT

(11) **EP 2 926 129 B1**
(45) Veröffentlichungstag und Bekanntmachung des Hinweises auf die Patenterteilung: **28.03.2018**
(21) Anmeldenummer: 13828974.9
(22) Anmeldetag: 02.12.2013
(51) Int. Cl.: G01N 21/3581, G01N 33/08

(54) **VERFAHREN ZUR GESCHLECHTERBESTIMMUNG VON VOGELEMBRYONEN IN VOGELEIERN DURCH DIE OPTISCH UNDURCHSICHTIGE EISCHALE HINDURCH**
METHOD FOR DETERMINING THE SEX OF BIRD EMBRYOS IN OVO THROUGH THE OPTICALLY IMPENETRABLE SHELL
PROCÉDÉ POUR DÉTERMINER LE SEX D'UN EMBRYON DANS UN OEUF À TRAVERS LA COQUILLE OPTIQUEMENT OPAQUE

(30) Priorität: 03.12.2012 DE 102012023947
(43) Veröffentlichungstag der Anmeldung: 07.10.2015
(73) Patentinhaber: Leibniz-Institut für Photonische Technologien e.V., 07745 Jena (DE)
(72) Erfinder: MAY, Torsten, 99510 Kleinromstedt (DE); WEBER, Karina, 07743 Jena (DE); POPP, Jürgen, 07751 Jena (DE)
(74) Vertreter: Donath, Dirk
(86) Internationale Anmeldenummer: PCT/DE2013/000784
(87) Internationale Veröffentlichungsnummer: WO 2014/086335

(56) Entgegenhaltungen:
- WO-A2-2004/023136
- WO-A2-2010/150265
- DE-A1-102007 013 107
- DE-B3-102010 006 161
- US-A- 5 504 572
- US-A1- 2002 075 476

## Beschreibung

Die Erfindung betrifft ein Verfahren und eine Anordnung zur nichtinvasiven, zerstörungsfreien Identifikation molekülspezifischer und/oder biologischer Eigenschaften einer inneren Struktur eines biologischen Untersuchungsobjektes durch eine optisch undurchlässige Barriere hindurch gemäß der Gattung der Patentansprüche, in Form eines Verfahrens und einer Vorrichtung zur Geschlechtsbestimmung von Vogelembryonen in ovo, bei welchem die Eihüllen der zu untersuchenden Vogeleier nicht eröffnet werden müssen.
Die Geschlechtsbestimmung von Lebewesen, insbesondere von Vögeln ist seit langen bekannt und kann auf verschiedenste Weise erfolgen.
Neben der rein äußeren Betrachtung der phänotypischen Merkmale von Lebewesen, wie bspw. Vögeln, kommen seit geraumer Zeit auch Hormon- und DNA- Untersuchungen zum Einsatz, um das Geschlecht dieser zu ermitteln.
So offenbart bspw. die DE 697 11 943 T2 ein Verfahren zur Geschlechtsbestimmung eines Vogels in ovo, bei welchem das Vorhandensein oder Fehlen eines geschlechtsspezifischen Hormons (Östrogen) in der extrazellulären Flüssigkeit des Vogeleis zur Ermittlung des Geschlechts verwendet wird.
Dabei wird eine Nadel oder ein Biosensor durch die eröffnete Eischale (Loch) hindurch in eine geeignete Zielgegend (bspw. allantonischer Sack) geführt, um eine Flüssigkeitsprobe aus dem Ei zu entnehmen oder in ovo den Hormongehalt zu messen (bspw. spektroskopisch).
Das Verfahren gemäß DE 697 11 943 T2 hat den Nachteil, dass die Geschlechtsbestimmung von Vogelembryonen, welche auch in ovo erfolgen kann, nicht zerstörungsfrei ist. Durch die Eröffnung der Eischale erfolgt eine Schädigung der natürlichen Schutzbarriere des Embryos (Schutz vor mikrobiellen, chemischen und mechanischen Einflüssen), was zu dessen Schädigung oder zu dessen Tod führen kann.

Aus der DE 10 2007 013 107 A1 ist ein Verfahren bekannt, durch welches das Geschlecht von Tieren auf möglichst einfache Weise, schnell, zuverlässig sowie ohne nennenswerte und auch aus Tierschutzgründen problematische Belastung der Tiere (beispielsweise durch Betäubung) bestimmbar ist.
Bei diesem Verfahren wird die DNA (bzw. Proteine) von Zellmaterial des geschlechtlich zu bestimmenden Vogels mit Licht untersucht und dabei die Molekül- bzw. Gerüstschwingungen der DNA (und der Proteine) gemessen.
Die zur Geschlechtsbestimmung wesentlichen Schwingungsbanden sind hierbei im Wellenzahlbereich unter 1800 cm⁻¹ zu finden.
Das durch die Molekül- bzw. Gerüstschwingungen der DNA entstehende Lichtspektrum wird erfasst und mit vorgegebenen Referenzspektren einer Datenbank verglichen. Aus diesem Vergleich wird eine auf dem DNA- Gehalt des Zellmaterials basierende Geschlechtszuordnung des Vogels getroffen.
Dieses Verfahren wird insbesondere angewendet, um das anhand äußerer Merkmale nicht oder unzureichend erkennbare Geschlecht von Vögeln, selbst in ovo, zu bestimmen.
Dazu wird aus der Federscheide von bereits aus dem Ei geschlüpften Vögeln als Zellmaterial Federpulpa gewonnen. Anschließend erfolgt eine Ableitung verschiedener UV- Resonanz- Raman- Spektren von der Federpulpa (Erfassung der Molekülschwingungen der DNA), welche bekannten unüberwachten oder überwachten Klassifizierungsmethoden zugeführt werden.
Alternativ zum Zellmaterial in Form von Federpulpa ist es gemäß der Offenbarung von DE 10 2007 013 107 A1 auch möglich, bei ungeschlüpften Vögeln Untersuchungslicht durch einen kleinen Zugang (ein Loch) durch die Eierschale unmittelbar auf die Keimscheibe als Zellmaterial zu fokussieren.

Dies hat jedoch den Nachteil, dass die Geschlechtsbestimmung von Vogelembryonen in ovo nicht zerstörungsfrei ist. Durch die Eröffnung der Eischale erfolgt eine Schädigung der natürlichen Schutzbarriere des Embryos (Schutz vor mikrobiellen, chemischen und mechansichen Einflüssen), was zu dessen Schädigung oder zu dessen Tod führen kann. Insbesondere bei der großtechnischen Aufzucht und Haltung von Nutzvögeln, wie bspw. Hühnern oder Puten, führt dieser Nachteil dazu, dass das Verfahren gemäß DE 10 2007 013 107 A1 in der Praxis nicht umsetzbar ist.

Die DE 10 2010 006 161 A1 offenbart ein Verfahren und eine Vorrichtung zur Bestimmung des Geschlechts von befruchteten und nicht bebrüteten Vogeleiern, wobei ein Ei eine feste Eischale, ein von der Eischale und weiteren Eihüllen umgebenen Eidotter und eine dem Eidotter zugeordnete Keimscheibe aufweist und wobei eine Sonde zur Messung eines Spektrums durch ein Loch der Eischale hindurch in Richtung zur Keimscheibe mit Keimscheibenzellen geführt wird.
Dabei werden folgenden Verfahrensschritte durchlaufen:
- Positionierung der Sonde im Bereich der Keimscheibe
- spektroskopische in- ovo Charakterisierung der Keimscheibenzellen und
- Erkennung des Geschlechts durch eine automatische Klassifizierung von Spektren.

Gemäß DE 10 2010 006 161 A1 wird als Sonde ein optischer Kristall eingesetzt, mit dem eine schnelle und rückwirkungsfreie Aufnahme eines Infrarot- und/oder Nahinfrarotspektrums bei Nutzung einer abgeschwächten Totalreflexion innerhalb des optischen Kristalls durch ein evaneszentes Feld im Bereich der Keimscheibe durchgeführt wird, wobei die Extinktion infolge einer spektralen Absorption von geschlechtsspezifischen Keimscheibenzellen erfolgt.
Dieses Verfahren hat ebenfalls den Nachteil, dass die Geschlechtsbestimmung von Vogelembryonen in ovo nicht zerstörungsfrei ist.

Hinzu kommt, dass dieses Verfahren den weiteren Nachteil besitzt, dass ein optischer Kristall verwendet werden muss.

Bekannt ist weiterhin, dass die Spektroskopie im Terahertz-Frequenzbereich zwischen 0,1THz und 10THz durchgeführt wird, wobei insbesondere im erweiterten Spektralbereich zwischen 0,025 und 100THz Spektren sowohl im Absorptions- als auch im Reflektionsverfahren gewonnen werden (siehe dazu bspw. GB 2,372,930 und GB 2,347,835).

Insbesondere ist es auch möglich, mittels zeitaufgelöster Ultrakurzpulsverfahren dreidimensionale (tomographische) Informationen über das Untersuchungsobjekt zu gewinnen (siehe dazu bspw. WO 2003042670 A1).

In US 7,612,341 wird dazu die spezielle Möglichkeit beschrieben, menschliches Gewebe in Reflektion spektral zu analysieren, um an Versuchspersonen einen Brustkrebs zu diagnostizieren.

Aus der DE 10 2007 031 959 A1 ist eine Terahertz- Videokamera bekannt, welche jedoch nicht zur Geschlechtsbestimmung eingesetzt werden kann.

Die nichtinvasive Bestimmung des Geschlechtes Vogelembryonen stellt ein derzeit noch ungelöstes Problem dar. Obwohl diverse Methoden prinzipiell in der Lage sind, genetisches Material hinsichtlich des Geschlechtes zu bestimmen, ist keine der etablierten Methoden in der Lage, diese Bestimmung durch eine ungeöffnete Eischale hindurch durchzuführen.

Die Aufgabe der Erfindung besteht somit darin, ein Verfahren und eine Anordnung anzugeben, welche die zuvor stehend genannten Nachteile des Standes der Technik vermeiden, in dem die nichtinvasive Identifikation chemischer und/oder biologischer Eigenschaften einer inneren Struktur eines Untersuchungsobjektes durch eine optisch undurchlässige Barriere des Untersuchungsobjektes hindurch, ohne die Entnahme von Probenmaterial direkt an der inneren Struktur durchgeführt wird, wobei das Untersuchungsobjekt in einem signifikanten Maß durchdrungen wird ohne dafür ionisierende Strahlung einzusetzen. Dabei darf die unter Umständen lebendige innere Struktur des Untersuchungsgegenstandes möglichst wenig beeinflusst werden bzw. erst recht nicht zerstört werden bzw. geschädigt werden.

Gelöst wird diese Aufgabe durch ein Verfahren gemäß dem 1. Patentanspruch. Vorteilhafte Ausgestaltungen der Erfindung sind in den nachgeordneten Ansprüchen angegeben.
Das Wesen der Erfindung besteht darin, dass vermittels Terahertz-Technologie durch eine optisch undurchsichtige Barriere eines Untersuchungsobjektes hindurch eine innere Struktur mit unterschiedlichen dielektrischen Eigenschaften mittels elektromagnetischer Volumenanalyse aufgeklärt wird und an Hand dieser aufgeklärten Strukturdaten des Untersuchungsobjektes eine gezielte Messung eines Spektrums eines gezielt ausgewählten Bereiches der aufzuklärenden Struktur mittels elektromagnetischer Spektralanalyse erfolgt, um ohne Probenentnahme aus dem Inneren des Untersuchungsobjektes Aussagen über molekülspezifische und/oder biologischen Eigenschaften der inneren Struktur zu treffen.

Die Voraussetzung für die Interpretation der an einem nicht weiter präparierten Untersuchungsobjekt gemessenen THz- Spektren ist die grundsätzliche Kenntnis über den generellen Aufbau des Objektes im Hinblick auf Schichtungen von Substanzen mit unterschiedlichen dielektrischen Eigenschaften, denn Grenzflächenreflektionen und Streuungen modifizieren das gewonnene Gesamtspektrum signifikant.

Um diese Vorkenntnis über im Objekt vorkommende Substanzen und deren mögliche Anordnung bzw. Schichtfolge zu erlangen, werden die Daten bzgl. der dielektrischen Eigenschaften des Objektes durch Labormessungen an präparierten Proben (d.h. an Proben ohne optisch undurchsichtige Barriere) im relevanten Spektralbereich gewonnen.

Insbesondere ist bei Proben in Form von Vogeleiern im Anwendungsfall für jedes Ei die Position der Keimscheibe, welche das zu analysierende genetische Material enthält, im Vorfeld der eigentlichen Messung zu ermitteln, um danach die spektrale Messung ortsgenau ohne zeitaufwendigen Justageprozess zerstörungsfrei am eigentlichen Untersuchungsobjekt durchzuführen.

Unter Benutzung der Vorkenntnisse über die zu analysierenden molekülspezifischen und/oder biologischen Eigenschaften der inneren Struktur werden mittels eines zeitaufgelösten Verfahrens die Laufzeiten von THz- Reflexen, welche durch die verschiedenen Grenzflächen hervorgerufen werden, ausgewertet, um ein ortsgenaues Modell des realen Schichtaufbaus im Untersuchungsobjekt zu generieren.

Für diese Ermittlung des Volumenmodells vom Untersuchungsobjekt können alternativ auch THz- ähnliche Messverfahren in einem erweiterten Spektralbereich von 0,01 bis 1 THz verwendet werden. Da in diesem Frequenzbereich bspw. bei der Geschlechtsanalyse befruchteter Vogeleier keine spektralen Informationen des genetischen Materials gewonnen werden können, eignet sich dieser erweitere Spektralbereich jedoch lediglich zur Erkundung der ortgenauen Lage der inneren Struktur. Die eigentliche Analyse des genetischen Materials muss in diesem Fall nach der genauen Positionierung des Untersuchungsobjekts im THz- Bereich erfolgen.

Das Volumenmodell des Schichtaufbaus auf Basis bekannter Materialdaten dient somit als Referenz, um das gemessene integrale Spektrum der untersuchten Probe des Untersuchungsobjekts zu analysieren. Auf diese Art können im ermittelten Gesamtspektrum nicht zur eigentlichen Probe gehörende Anteile extrahiert bzw. bekannte Anteile quantifiziert werden.

Die eigentliche Messung des integralen Spektrums im THz- Bereich erfolgt durch die parallele Aufnahme von Signalen bei verschiedenen Frequenzen als Antwort auf eine breitbandige, möglichst homogene Beleuchtung nach dispersiver Zerlegung mittels einer Zeilenanordnung breitbandiger Detektoren.

Die Detektoren können dabei zur Erzielung maximaler Empfindlichkeit und damit verbundener minimaler Messzeit gekühlt werden.

Das erfindungsgemäße Messverfahren umfasst zwei unabhängige Messverfahren zur Aufbauanalyse (Ermittlung des Volumenmodells) und der Messung des integralen Spektrums (der eigentlichen Probe).

Die Ermittlung des Volumenmodells erfolgt durch eine Verfahren zur Strukturaufklärung durch eine optisch undurchsichtige Barriere eines biologischen Untersuchungsobjektes hindurch, bei dem eine innere Struktur mit unterschiedlichen dielektrischen Eigenschaften mittels elektromagnetischer Spektralanalyse aufgeklärt wird, in dem das Untersuchungsobjekt unter einem Array von Pulssendern und Empfängern positioniert wird, welche in einer Ebene angeordnet und daten- sowie informationsleitend mit einem Computersystem verbunden sind. Durch elektronische Pulssenderstrahlen werden elektromagnetische Pulse im Spektralbereich 0,01 bis 1 THz auf das positionierte Untersuchungsobjekt ausgesendet. Die von Untersuchungsobjekt ausgehende Strahlung wird von elektronischen Empfängern aufgenommen und dem Computer über daten- sowie informationsleitende Verbindungen für ein bildgebendes Verfahren zugeleitet, vermittels dessen die Bilddaten computergestützt automatisch ausgewertet und 3D- Raumkoordinaten zugeordnet werden, in dem diese Bilddaten mit den Daten einer Datenbank spektraler Materialparameter abgeglichen werden, die zuvor durch Labormessungen der dielektrischen Eigenschaften an Proben vom Untersuchungsobjekt ohne optisch undurchsichtige Barriere im relevanten Spektralbereich gewonnen wurden.

Die 3D- Raumkoordinaten, welche bildlich dargestellt werden können, steuern eine Positioniereinheit in den Raumrichtungen X-Y-Z.

Die Messung des integralen Spektrums der eigendlichen Probe erfolgt durch ein Verfahren zur Strukturaufklärung durch eine optisch undurchsichtige Barriere eines biologischen Untersuchungsobjektes hindurch, in dem das Untersuchungsobjekt durch die Positioniereinheit in einem weiteren Verfahrensschritt so im Raum positioniert wird, dass die von einer Strahlungsquelle ausgesendete breitbandigen, homogenen Beleuchtung einer Frequenz im Bereich von 0, 1 bis 10 THz die Raumkoordinaten (x,y,z) einer molekülspezifisch und/oder biologisch aufzuklärende inneren Struktur durchdringt und die dabei ausgesendeten THz- Spektren durch einen Zeilenempfänger aufgenommen werden. Alternativ dazu ist auch möglich, dass gegenüber dem Untersuchungsobjekt ein durch die Positioniereinheit im Raum positionierter Messkopf mit Strahlungsquelle in einem weiteren Verfahrensschritt so angeordnet wird, dass die von der Strahlungsquelle ausgesendete breitbandigen, homogenen Beleuchtung einer Frequenz im Bereich von 0, 1 bis 10 THz die Raumkoordinaten (x,y,z) eine chemisch und/oder biologisch aufzuklärende inneren Struktur durchdringt und die dabei ausgesendeten THz- Spektren durch einen Zeilenempfänger aufgenommen werden.
Folgende Schritte sind dabei durchzuführen:

### 1) Aufbau der Materialparameter-Datenbank

Vor der Durchführung der eigentlichen Messung sind die Materialparameter der Vogelei-Substanzen unter Laborbedingungen zu ermitteln. Dazu können aus der jeweils zu untersuchenden Charge Stichproben entnommen werden, welche neben möglichst exakten Parametern auch Angaben über die Schwankungsbreite der jeweiligen Charge liefern.

### 2) Strukturanalyse

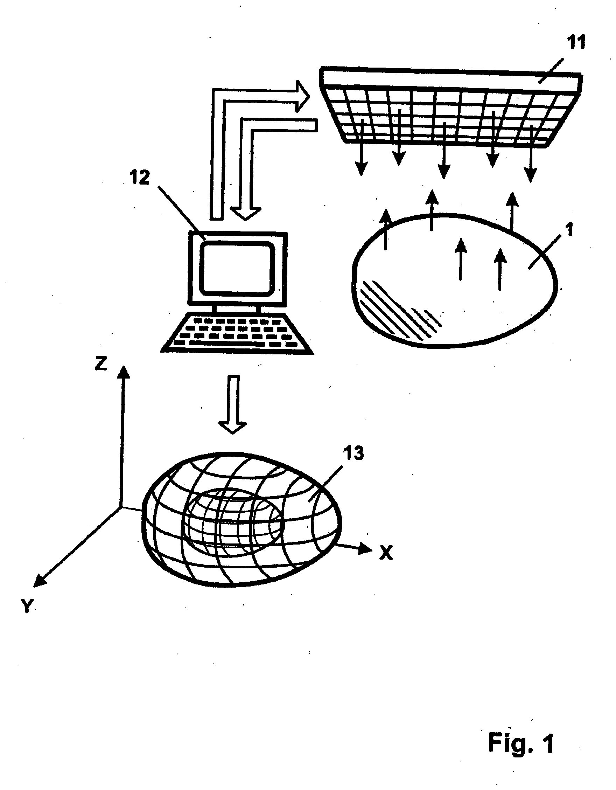

Jedes Untersuchungsobjekt mit optisch undurchsichtiger Barriere, d.h. ein Vogelei durchläuft eine Messstation mit einem bildgebenden Verfahren auf Basis elektromagnetischer Pulse im Spektralbereich 0,01 - 1 THz, wodurch der genaue Schichtaufbau des Untersuchungsobjektes, d.h. des Vogeleis sowie die aktuelle Position der chemisch und/oder biologisch aufzuklärenden Struktur (d.h. die Keimscheibe) ermittelt werden (Abbildung 1). Die Bilddaten werden automatisch ausgewertet und dienen im nächsten Schritt zur genauen Positionierung des Messkopfes in Bezug auf die chemisch und/oder biologisch aufzuklärende Struktur für die eigentliche Spektralanalyse.

### 3) Spektralmessung

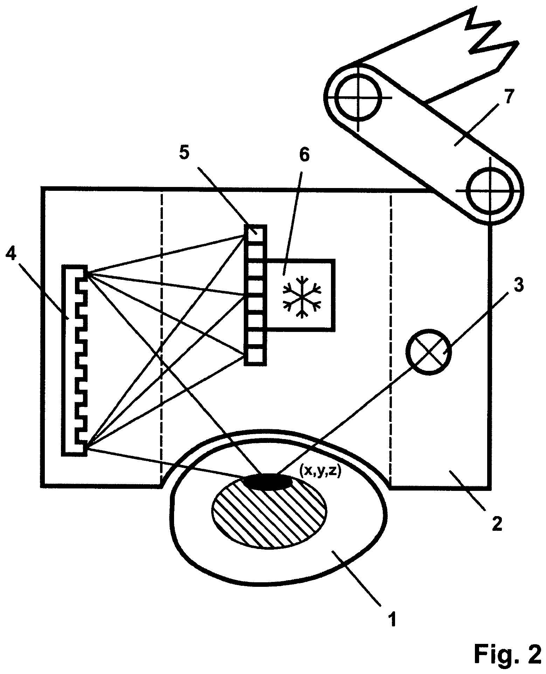

Zur Messung des integralen Spektrums wird ein Messkopf (Abbildung 2), bestehend aus einer leistungsstarken Beleuchtungsquelle im Spektralbereich 0,1 - 10 THz und eine kompakten Spektrometer robotisch gegenüber der aufzuklärende Struktur so positioniert, dass die zuvor lokalisierte Struktur die Keimscheibe in einem Vogelei) mit möglichst großem Querschnitt im Strahlfokus liegt. Im Ergebnis der spektroskopischen Messung im THz- Bereich entsteht ein integrales Spektrum, welches aus der Überlagerung der Spektren aller durchstrahlten Komponenten der Struktur der Keimscheibe) besteht.

### 4) Auswertung

Zur Extraktion des relevanten Einzelspektrums der Struktur (d.h. des genetische Materials der Keimscheibe) werden auf Basis der voruntersuchten Materialparameter und des Volumenmodells Spektren der Einzelkomponenten des Untersuchungsobjektes (d.h. des Vogeleis) berechnet und vom gemessenen integralen Spektrum subtrahiert. Auf Basis adaptiver Software-Algorithmen wird das Modell innerhalb einer Charge verfeinert. Zum Ende der Messung einer Charge (>1000 Untersuchungsobjekte, bspw. Vogeleier) wird das Datenaufkommen ausgewertet und mit statistischen Methoden unter der Voraussetzung zu erwartender spezifischer (d.h. geschlechtsspezifischer) Merkmale analysiert.

Die zuvor stehenden zwei Messverfahren zur Ermittlung des Volumenmodelles mit anschließender Messung des integralen Signals der eigentlichen Probe können zur Geschlechterbestimmung von Vogelembryonen eingesetzt werden, in dem die vom Zeilenempfänger erfassten THz- Absorbtionsspektren mit Referenzspektren rechnergestützt vermittels chemometrisch-mathematisch-statistischen Klassifizierungsmethoden der Datenanalyse verglichen werden, um das Geschlecht zu bestimmen.

Auf Basis dieser Untersuchung ist es möglich, das Geschlecht von Vogelembryonen mit einer für die Praxis relevanten Geschwindigkeit (industrielle Anwendung) durch die ungeöffnete Eischale sowie die Eihaut und das Eiklar hindurch anhand der Quantifizierung der THz-Spektralsignatur der DNA der Keimscheibe zu bestimmen.
Dazu wird das Zellmaterial der Keimscheibe (die DNA) des geschlechtlich zu bestimmenden Vogelembryos mit THz- Strahlung durch die intakte Eihülle hindurch untersucht, in dem die Molekülschwingungen gemessen werden, das Spektrum der Molekülschwingungen erfasst und mit vorgegebenen, geschlechtsspezifischen DNA- Strukturen der zu untersuchenden Vogelart repräsentierenden Referenzspektren verglichen wird und aus diesem Spektralvergleich eine Geschlechtszuordnung des Vogelembryos erfolgt.
Der Vergleich der erfassten THz- Absorbtionsspektren mit den Referenzspektren erfolgt dabei rechnergestützt mit chemometrisch-mathematisch-statistischen Methoden zur Datenanalyse an Hand bereits bekannter Klassifizierungsmethoden.

Für die Durchführung des erfindungsgemäßen THz- Untersuchungsverfahrens wird eine Anordnung aus leistungsstarker THz- Quelle und höchstempfindlicher Detektionstechnik verwendet, die für die Strukturanalyse (Verfahrensschritt 2) aus einem Array von Pulssendern und Empfängern besteht, welche in einer Ebene angeordnet ist und Daten- sowie Informationsleitend mit einem Computersystem verbunden ist, wobei ein optisch undurchsichtiges Untersuchungsobjekt unter dem Array positionierbar ist.
Für die Durchführung des erfindungsgemäßen THz- Untersuchungsverfahrens wird eine Anordnung aus leistungsstarker THz- Quelle und höchstempfindlicher Detektionstechnik verwendet, die für die Spektralmessung (Verfahrensschritt 3) aus einem Messkopf, einer Beleuchtungsquelle, einem diffraktivem Element, einem Zeilenempfänger, einem Kühler (welcher optional ist) und einer Positioniervorrichtung, vermittels derer der Messkopf gegenüber einer markierten Messposition eines Untersuchungskörpers positionierbar ist, besteht.
Die Erfindung wird nachfolgend an Hand eines Ausführungsbeispiels und der Figuren näher erläutert. Dabei zeigen:
- Fig. 1:: eine schematische Darstellung einer Ausführungsform einer Anordnung zur Strukturaufklärung (Verfahrensschritt 2)
- Fig. 2:: eine schematische Darstellung einer Ausführungsform einer Anordnung zur Messung des Spektrums (Verfahrensschritt 3) und
- Fig. 3:: eine schematische Übersichtsdarstellung der Verknüpfung von Verfahrensschritt 2 und 3.

Die in der Fig. 1 dargestellte Ausführungsform der Anordnung umfasst eine höchstempfindliche aktive Detektionstechnik in Form eines Arrays (11) von Pulssendern und Empfängern, welche in einer Ebene angeordnet sind und Daten- sowie Informationsleitend mit einem Computersystem (12) verbunden sind, wobei ein optisch undurchsichtiges Untersuchungsobjekt unter dem Array positionierbar ist.
Die in Fig. 2 dargestellte Ausführungsform der Anordnung umfasst eine leistungsstarke THz- Quelle, höchstempfindliche Detektionstechnik in Form eines Messkopfes (2), welcher eine Beleuchtungsquelle (3), ein diffraktives Element (4), einen Zeilenempfänger (5) und einem Kühler (6) [welcher optional ist] umschließt, und eine Positioniervorrichtung (7), vermittels derer der Messkopf (2) gegenüber einer markierten Messposition eines Untersuchungskörpers (1), [d.h. der Keimscheibe eines Vogelembryos] in X-Y-Z- Richtung 3- dimensional positionierbar ist. Während des Messvorgangs verläuft ein Messstrahl von der Beleuchtungsquelle (3) auf den Untersuchungskörper (1) in Form der Keimscheibe eines Vogelembryos. Von diesem Untersuchungskörpers (1) verläuft der Strahlengang weiter über das diffraktive Element (4) zum Zeilenempfänger (5).

### Positionierung einer Keimscheibe eines Vogelembryos zur Vorbereitung einer nachfolgenden THz- absorbtionsspektroskopischen Untersuchung dieser Keimscheibe mittels der in Figur 1 dargestellten Anordnung zur Strukturaufklärung

Vor der Durchführung der eigentlichen Positionierung und Messung sind die Materialparameter der Vogelei-Substanzen unter Laborbedingungen zu ermitteln. Dazu können aus der jeweils zu untersuchenden Charge von Vogeleiern Stichproben entnommen werden, welche neben möglichst exakten Parametern auch Angaben über die Schwankungsbreite der jeweiligen Charge liefern.

Die Positionierung des Untersuchungsobjekts wird an einem Vogelei, welches eine Keimscheibe eines ein Vogelembryos enthält, durchgeführt.
Dazu wird das Vogelei (1) auf dem Halter (8) fixiert, so dass seine Position gegenüber dem Halter (8) nicht veränderbar ist.
Nach dem Fixieren wird das Vogelei (1) durch ein Array (11) mit elektronischen Pulssenderstrahlen mit einer Frequenz im Bereich von 0,01 bis 0,1 THz zur Absorption bestrahlt und dabei gleichzeitig über das Array mit elektronischen Empfängern die von Vogelei (1) ausgesendete Strahlung vermessen.
Die durch das Array (11) mit Empfängern ermittelten Messdaten werden an das Computersystem (12) übermittelt, welches mittels eines zeitaufgelösten Verfahrens die Laufzeiten von einzelnen THz-Reflexen an verschiedenen Grenzflächen auswertet und an Hand dieser Daten ein rekonstruiertes Volumenmodell des realen Schichtaufbaus des Vogel eis (1) erstellt.
Dabei werden die Raumkoordinaten (x,y,z) der Keimscheibe ermittelt und als markierte Messposition im Computersystem (12) gespeichert.

### THz- absorbtionsspektroskopischen Untersuchung der Keimscheibe zur Geschlechtszuordnung des Vogelembryos mittels der in Figur 2 dargestellten Anordnung zur Messung des Spektrums

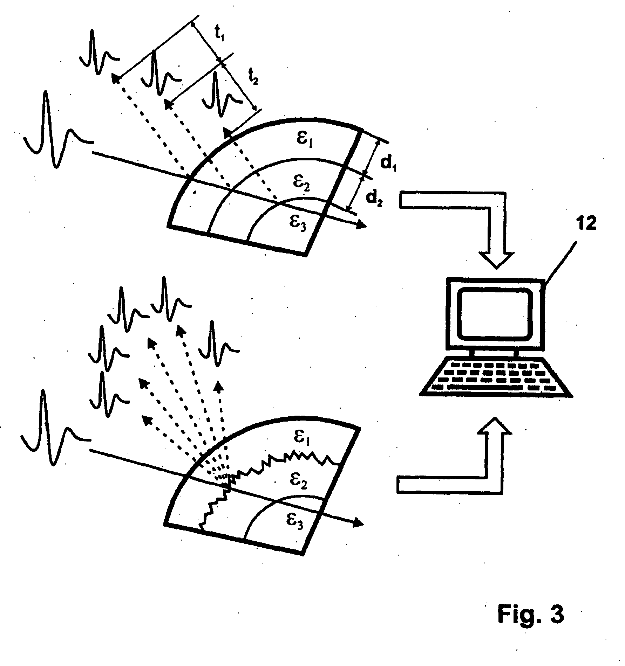

Die THz- absorbtionsspektroskopischen Untersuchungen werden exemplarisch an einer vorher positionierten Keimscheibe durchgeführt. Dazu dient die im Computersystem (12) gespeicherte markierte Messposition der Raumkoordinaten (x,y,z) der Keimscheibe für die robotische Positionierung des Messkopfes (2) über dem Vogelei (1), in dem der Messkopf (2) durch die Positioniervorrichtung (7) vermittels der Positionierdaten des Computersystems (12), der datenleitend mit der Positioniervorrichtung (7) verbunden ist, über dem Vogelei (1) bewegt wird, bis der von der Beleuchtungsquelle (3) entsendete Messstrahl in Form einer breitbandigen, möglichst homogenen Beleuchtung einer Frequenz im Bereich von 1 bis 10 THz auf die markierte Messposition der Raumkoordinaten (x,y,z) der Keimscheibe mit möglichst großen Querschnitt trifft und die von dieser Messposition ausgesendete Strahlung über das diffraktive Element (4) auf den Zeilenempfänger (5) als breitbandigen Detektor trifft und dort ein integralen Spektrum erzeugt (siehe dazu Figur 3: schematische Übersichtsdarstellung der Verknüpfung).
Das diffraktive Element (4) kann dabei als mikrofabriziertes Beugungsgitter ausgeführt sein, welches das von Vogelei (1)reflektierte Licht spektrol zerlegt und auf die Zeilenanordnung von gekühlten sipraleitenden Sensoren des Zeilenempfängers (5) abbildet.

Als Referenz für die auf dem Zeilenempfänger (5) auftreffenden Spektraldaten wird ein Modell des Schichtaufbaus bekannter Spektraldaten einer Keimscheibe verwendet, wobei vermittels des Computersystems (12) das gemessene integrale Spektrum der untersuchten Messposition der Raumkoordinaten (x,y,z) der Keimscheibe analysiert wird.
Zur Extraktion des relevanten Einzelspektrums des genetische Materials der Keimscheibe werden dabei vermittels des Computersystems (12) auf Basis der voruntersuchten Materialparameter und des Volumenmodells Spektren der Einzelkomponenten des Vogeleis berechnet und vom gemessenen integralen Spektrum subtrahiert. Auf Basis adaptiver Software-Algorithmen wird das Modell innerhalb einer Charge verfeinert. Zum Ende der Messung einer Charge (>1000 Vogeleier) wird das Datenaufkommen ausgewertet und mit statistischen Methoden unter der Voraussetzung zu erwartender geschlechtsspezifischer Merkmale analysiert.
Der Vergleich der erfassten, aus dem integralen Spektrum berechneten geschlechtsspezifischen THz- Absorbtionsspektren mit den vorbekannten Referenzspektren "männlich" oder "weiblich" erfolgt dabei vermittels des Computersystems (12) rechnergestützt mit chemometrisch-mathematisch-statistischen Methoden zur Datenanalyse an Hand bereits bekannter Klassifizierungsmethoden.
Der Vorteil des erfindungsgemäßen Verfahrens und der erfindungsgemäßen Vorrichtung besteht darin, dass eine nichtinvasive Identifikation chemischer und/oder biologischer Eigenschaften einer inneren Struktur eines Untersuchungsobjektes durch eine optisch undurchlässige Barriere hindurch möglich ist, ohne die Barriere zu zerstören.

Dies ist besonders vorteilhaft, wenn das Untersuchungsobjekt ein Vogelei ist, bei welchem die Eihüllen nicht eröffnet werden soll, um eine Geschlechtsbestimmung des vom Ei umschlossenen Vogelembryos in ovo ohne eine durch die Eischale hindurchzuführende Sonde durchzuführen.
Alle in der Beschreibung und den nachfolgenden Ansprüchen dargestellten Merkmale können sowohl einzeln, als auch in beliebiger Kombination miteinander, erfindungswesentlich sein.

### Bezugszeichenliste

- 1: - Untersuchungsobjekt (bspw. Vogelei)
- 11: - Array von Pulssendern und Empfängern
- 12: - optisch undurchsichtige Hülle des Untersuchungsobjekt (bspw. Schale des Vogeleis)
- 13: - rekonstruiertes Volumenmodell
- 14: - PC
- 2: - Messkopf
- 3: - Beleuchtungsquelle
- 4: - diffraktives Element
- 5: - Zeilenempfänger
- 6: - Kühler (optional)
- 7: - Positioniervorrichtung

- x,y,z: - Raumkoordinaten
- X-Y-Z: - Richtungen des 3-D- Raumes

## Patentansprüche

1. Verfahren zur Geschlechterbestimmung von Vogelembryonen in Vogeleiern durch die optisch undurchsichtige Eischale hindurch, bei dem das zu analysierende genetische Material in der Keimscheibe mittels elektromagnetischer Spektralanalyse aufgeklärt wird, **dadurch gekennzeichnet, dass**
- in einem 1. Schritt das Vogelei unter einem Array von Pulssendern und Empfängern, welche in einer Ebene angeordnet und daten- sowie informationsleitend mit einem Computersystem verbunden sind, angeordnet ist, wobei durch die Pulssender elektromagnetische Pulse im Spektralbereich 0,01 bis 1 THz durch die optisch undurchsichtige Eischale hindurch auf das Vogelei ausgesendet werden und gleichzeitig die von dem Vogelei zurückgeworfene, durch die optisch undurchsichtige Eischale hindurch gehende Strahlung vermessen und dem Computersystem über daten- sowie informationsleitende Verbindungen übermittelt wird, wobei mittels des Computers ein rekonstruiertes Volumenmodell mit 3D-Raumkoordinaten (x,y,z) des Vogeleis erstellt wird, und anschließend
- in einem 2. Schritt ein breitbandiger elektromagnetischer Sender an Hand der im ersten Schritt ermittelten 3D-Raumkoordinaten (x,y,z) durch eine Positioniervorrichtung robotisch gegenüber der genetisch aufzuklärenden Keimscheibe so positioniert wird, dass durch die optisch undurchsichtige Eischale hindurch elektromagnetische Wellen im Spektralbereich 0,01 bis 10 THz auf die genetisch aufzuklärende Keimscheibe ausgesendet werden und die von der Keimscheibe ausgehende, durch die optisch undurchsichtige Eischale hindurch gehende Strahlung von einem Spektralempfänger aufgenommen und dem Computersystem über die daten- sowie informationsleitenden Verbindungen zugeleitet wird, wobei das mit dem Empfänger erfasste integrale Spektrum der genetisch aufzuklärenden Keimscheibe computergestützt automatisch ausgewertet und mit den Daten einer Datenbank spektraler Materialparameter abgeglichen wird, die zuvor durch Labormessungen der dielektrischen Eigenschaften an Proben von Vogeleiern ohne optisch undurchsichtige Eischale im relevanten Spektralbereich gewonnen wurden.

2. Verfahren zur Geschlechterbestimmung von Vogelembryonen in Vogeleiern durch die optisch undurchsichtige Eischale hindurch gemäß Anspruch 1, **dadurch gekennzeichnet, dass** die 3D-Raumkoordinaten (x,y,z) bildlich dargestellt werden.

3. Verfahren zur Geschlechterbestimmung von Vogelembryonen in Vogeleiern durch die optisch undurchsichtige Eischale hindurch gemäß Anspruch 1, **dadurch gekennzeichnet, dass** die Positioniereinheit in den Raumrichtungen X-Y-Z gesteuert wird.

4. Verfahren zur Geschlechterbestimmung von Vogelembryonen in Vogeleiern durch die optisch undurchsichtige Eischale hindurch gemäß Anspruch 3, **dadurch gekennzeichnet, dass** das zu untersuchende Vogelei durch die Positioniereinheit in dem 1. Verfahrensschritt so im Raum positioniert wird, dass die von einer Strahlungsquelle ausgesendete breitbandigen, homogenen Beleuchtung einer Frequenz im Bereich von 0, 1 bis 10 THz die Raumkoordinaten (x,y,z) in dem 2. Verfahrensschritt die genetisch aufzuklärende Keimscheibe des Vogeleis durchdringt und die dabei ausgesendeten THz- Spektren durch einen Zeilenempfänger aufgenommen werden.

5. Verfahren zur Geschlechterbestimmung von Vogelembryonen in Vogeleiern durch die optisch undurchsichtige Eischale hindurch gemäß Anspruch 3, **dadurch gekennzeichnet, dass** gegenüber dem Vogelei ein durch die Positioniereinheit im Raum positionierter Messkopf mit Strahlungsquelle in dem 1. Verfahrensschritt so angeordnet wird, dass die von der Strahlungsquelle ausgesendete breitbandige, homogene Beleuchtung einer Frequenz im Bereich von 0, 1 bis 10 THz die Raumkoordinaten (x,y,z) in dem zweiten Verfahrensschritt die genetisch aufzuklärende Keimscheibe durchdringt und die dabei ausgesendeten THz- Spektren durch einen Zeilenempfänger aufgenommen werden.

6. Verwendung eines Verfahrens gemäß einem oder mehrerer der voran stehenden Ansprüche 1 bis 5 zur Geschlechterbestimmung eines Vogelembryos in einem völlig geschlossenen Ei, bei der mittels des 1. Verfahrensschritts ein bestimmter Bereich der genetisch aufzuklärenden Keimscheibe eines Vogelembryos ermittelt wird, dessen Raumkoordinaten (x,y,z) zur Markierung der Messposition im Computersystem gespeichert werden und zur Ansteuerung des Messkopfes für die anschließende THz- absorptionsspektroskopische Untersuchung verwendet werden, wobei in dem 2. Verfahrensschritt das erfasste integrale Spektrum der Messposition mit Referenzspektren computergestützt vermittels chemometrisch-mathematisch- statistischen Klassifizierungsmethoden der Datenanalyse verglichen wird, um das Geschlecht von Vogelembryonen zu bestimmen.

## Claims

1. A method for the determination of the gender of bird embryos in bird eggs through the optically opaque eggshell, in which the genetic material in the germ disc to be analysed is clarified by electromagnetic spectral analysis, **characterized in that**
- in a first step, the bird's egg is positioned under an array of pulse transmitters and receivers which are arranged in one plane and connected to a computer system for data and information transfer, wherein electromagnetic pulses in the spectral range from 0.01 to 1 THz are transmitted by the pulse transmitters through the optically opaque eggshell to the bird's egg, and simultaneously the radiation reflected by the bird's egg and passing the optically opaque eggshell is measured and transferred to the computer system via data and information transmission connections, wherein a reconstructed volume model of the bird's egg with 3D spatial coordinates (x,y,z) is created by the computer, and afterwards
- in a second step, on the basis of the 3D spatial coordinates (x,y,z) determined in the first step, a positioning device robotically positons a broadband electromagnetic transmitter relative to a measurement area of the test object to be examined in such a way that electromagnetic waves in the spectral range from 0.01 to 10 THz are emitted through the optically opaque barrier to the measurement area of the previously positioned test object and the radiation emitted by the measurement area and passing through the optically opaque barrier is received by a spectral receiver and sent to the computer system via data and information transmission connections, wherein the integral spectrum of the measurement area recorded by the receiver is automatically analysed by a computer and compared with the data of a database of spectral material parameters which were previously obtained by laboratory measurements of the dielectric properties of samples of bird eggs in the relevant spectral range without optically opaque eggshells.

2. The method for the determination of the gender of bird embryos in bird eggs through the optically opaque eggshell according to claim 1, **characterized in that** the 3D spatial coordinates (x,y,z) are represented graphically.

3. The method for the determination of the gender of bird embryos in bird eggs through the optically opaque eggshell according to claim 1, **characterized in that** the positioning device is controlled in the spatial directions X-Y-Z.

4. The method for the determination of the gender of bird embryos in bird eggs through the optically opaque eggshell according to claim 3, **characterized in that** in the first process step the bird's egg to be examined is positioned by the positioning device in the space in such a way that in the second process step the broadband, homogeneous illumination with a frequency in the range from 0.1 to 10 THz emitted by a radiation source penetrates the germ disc of the bird's egg, which is to be genetically analysed, in the area of the spatial coordinates (x,y,z) determined in the first step, and the THz spectra emitted thereby are received by a line receiver.

5. The method for the determination of the gender of bird embryos in bird eggs through the optically opaque eggshell according to claim 3, **characterized in that** in the first step a gauge head is positioned by the positioning device relative to the bird's egg in such a way that in the second process step the broadband, homogenous illumination with a frequency ranging from 0.1 to 10 THz emitted by a radiation source penetrates a germ disc, which is to be chemically and/or biologically analysed, in the area of the spatial coordinates (x,y,z) determined in the first step, and the THz spectra emitted thereby are received by a line receiver.

6. Use of a method according to one or several of the previous claims 1 to 5 for the determination of the gender of a bird's embryo in a completely closed egg, in which in the first process step a specific area of the germ disc of a bird's embryo to be genetically analysed is determined, its spatial coordinates (x,y,z) are stored in the computer system to mark the measurement position and they are used to trigger the gauge head for the subsequent THz absorption-spectroscopy investigation, wherein in the second process step the recorded integral spectrum of the measurement position is compared with reference spectra in a computerised procedure by applying chemometric-mathematic-statistic classification methods of data analysis to determine the gender of bird embryos.

## Revendications

1. Procédé pour déterminer le sexe d'un embryon dans un oeuf à travers la coquille optiquement opaque, pendant lequel le matériel génétique à analyser dans le disque germinatif est éclairé au moyen de l'analyse spectrale électromagnétique, est **caractérisé en ce que**
- dans une première étape, l'oeuf aviaire est disposé sous un ensemble d'émetteurs impulsionnels et récepteurs qui sont situés dans un même plan et qui sont connectés à un système informatique pour transmettre des données et informations et les émetteurs impulsionnels émettent des impulsions électromagnétiques dans la gamme spectrale de 0,01 à 1 THz à travers la coquille optiquement opaque sur l'oeuf aviaire et simultanément le rayonnement reflété par l'oeuf aviaire, mais traversant la coquille optiquement opaque est mesuré et transmis au système informatique par des lignes de données et informations et au moyen de l'ordinateur un modèle volumique reconstruit avec des coordonnées spatiales 3D (x,y,z) de l'oeuf aviaire est créé et
- dans une deuxième étape un émetteur électromagnétique à large bande est positionné par un dispositif de positionnement au moyen des coordonnées spatiales 3D (x,y,z) déterminées pendant la première étape de façon robotique sur le côté opposé d'une gamme de mesure à examiner de l'objet de recherche de sorte que des ondes électromagnétiques dans la gamme spectrale de 0,01 à 10 THz soient émis à travers de la barrière optiquement opaque sur la gamme de mesure à examiner de l'objet de recherche positionné et le rayonnement émit par la gamme de mesure, traversant la barrière optiquement opaque soit capté par un récepteur spectral et transmis au système informatique par les lignes de données et informations, et le spectre intégral de la gamme de mesure capté par le récepteur soit évalué automatiquement et comparé avec les données d'une base de données comportant des paramètres matériels gagnées auparavant grâce aux mesures en laboratoire des propriétés diélectriques sur des échantillons des oeufs aviaires sans coquille optiquement opaque dans la gamme spectrale pertinente.

2. Procédé pour déterminer le sexe d'un embryon dans un oeuf à travers la coquille optiquement opaque suivant la revendication 1 est **caractérisé en ce que** les coordonnées spatiales 3D (x,y,z) sont représentées visuellement.

3. Procédé pour déterminer le sexe d'un embryon dans un oeuf à travers la coquille optiquement opaque suivant la revendication 1 est **caractérisé en ce que** l'unité de positionnement est contrôlée par les directions spatiales X-Y-Z.

4. Procédé pour déterminer le sexe d'un embryon dans un oeuf à travers la coquille optiquement opaque suivant la revendication 3 est **caractérisé en ce que** - pendant la première étape de procédé - l'oeuf aviaire à examiner est positionné par l'unité de positionnement en espace de sorte que l'éclairage homogène à large bande émis par une source de rayonnement avec une fréquence dans la gamme de 0, 1 à 10 THz pour les coordonnées spatiales (x,y,z) pénètre dans la deuxième étape de procédé le disque germinatif de l'oeuf aviaire à éclairer génétiquement et les spectres THz émis soient captés par un récepteur à lignes.

5. Procédé pour déterminer le sexe d'un embryon dans un oeuf à travers la coquille optiquement opaque suivant la revendication 3 est **caractérisé en ce qu'**une tête de mesure positionnée en espace par l'unité de positionnement est placée sur le côté opposé de l'oeuf aviaire dans la première étape de procédé de sorte que l'éclairage homogène à large bande émis par une source de rayonnement avec une fréquence dans la gamme de 0, 1 à 10 THz pour les coordonnées spatiales (x,y,z) pénètre dans la deuxième étape de procédé le disque germinatif de l'oeuf aviaire à éclairer chimiquement et/ou biologiquement et les spectres THz émis soient captés par un récepteur à lignes.

6. Utilisation d'un procédé suivant une ou plusieurs des revendications précédentes 1 à 5 pour déterminer le sexe d'un embryon aviaire dans un oeuf complètement fermé, pendant laquelle une certaine gamme du disque germinatif d'un embryon aviaire à éclairer génétiquement est définie dans la première étape de procédé, dont les coordonnées spatiales (x,y,z) sont enregistrées dans le système informatique pour marquer la position de mesure et utilisées pour commander la tête de mesure pour l'examen ultérieur par spectroscopie par absorption THz et, pendant la deuxième étape de procédé, le spectre intégral capté de la position de mesure est comparé à l'aide du système informatique avec des spectres de référence au moyen des méthodes de classification chimiométriques-mathématiques-statistiques de l'analyse des données pour déterminer le sexe des embryons aviaires.
